(19) Europäisches Patentamt / European Patent Office / Office européen des brevets

(11) **EP 1 929 006 B1**

(12) **EUROPEAN PATENT SPECIFICATION**

(45) Date of publication and mention of the grant of the patent:
**03.12.2014 Bulletin 2014/49**

(21) Application number: **06811475.0**

(22) Date of filing: **02.10.2006**

(51) Int Cl.:
*C12N 9/64* (2006.01)     *C12N 5/07* (2010.01)

(86) International application number:
**PCT/JP2006/320148**

(87) International publication number:
**WO 2007/037561 (05.04.2007 Gazette 2007/14)**

(54) **ACTIVATING AGENT OF STEM CELLS AND/OR PROGENITOR CELLS**

AKTIVIERUNGSMITTEL FÜR STAMMZELLEN UND/ODER VORLÄUFERZELLEN

AGENT D'ACTIVATION DES CELLULES SOUCHES ET/OU DES CELLULES PROGENITEURS

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.09.2005 JP 2005288436**

(43) Date of publication of application:
**11.06.2008 Bulletin 2008/24**

(73) Proprietor: **Tobishi Pharmaceutical Co., Ltd.**
**Tokyo**
**1000006 (JP)**

(72) Inventors:
• **SENGA, Hirobumi**
  **Tokyo 1050022 (JP)**
• **HAN, Zhongchao**
  **Tianjin Economic-Technological Development Area (TEDA)**
  **Tianjin (CN)**
• **CAI, Dingfang**
  **Shanghai (CN)**
• **LI, Li**
  **Tianjin 300121 (CN)**

(74) Representative: **Vossius & Partner**
**Siebertstrasse 4**
**81675 München (DE)**

(56) References cited:
**WO-A-2005/090550**

• **ITOH N ET AL: "MOLECULAR CLONING AND SEQUENCE ANALYSIS OF CDNA FOR BATROXOBIN, A THROMBIN-LIKE SNAKE VENOM ENZYME" JOURNAL OF BIOLOGICAL CHEMISTRY, AMERICAN SOCIETY OF BIOLOCHEMICAL BIOLOGISTS, BIRMINGHAM,, US, vol. 262, no. 7, 5 March 1987 (1987-03-05), pages 3132-3135, XP002019838 ISSN: 0021-9258**
• **RAJENDRA WUDAYAGIRI ET AL: "Neuroprotection and peptide toxins" BRAIN RESEARCH REVIEWS, vol. 45, no. 2, May 2004 (2004-05), pages 125-141, XP002410181 ISSN: 0165-0173**
• **BELL WILLIAM R JR: "Defibrinogenating enzymes" DRUGS, vol. 54, no. SUPPL. 3, 1997, pages 18-31, XP009075819 ISSN: 0012-6667**
• **TERAMOTO TETSUYUKI ET AL: "EGF amplifies the replacement of parvalbumin-expressing striatal interneurons after ischemia." THE JOURNAL OF CLINICAL INVESTIGATION. APR 2003, vol. 111, no. 8, April 2003 (2003-04), pages 1125-1132, XP002410183 ISSN: 0021-9738**
• **LORENZINI ET AL.: "349 Granulocyte colony stimulating factor (G-CSF) administration in cirrhotic patients: A phase II study evaluating tolerability profile and mobilization of haematopoietic stem cells (HSCS)" JOURNAL OF HEPATOLOGY, MUNKSGAARD INTERNATIONAL PUBLISHERS, COPENHAGEN, DK, vol. 42, April 2005 (2005-04), page 129, XP005060160 ISSN: 0168-8278**

**Description**

TECHNICAL FIELD

The present invention relates to an activating agent of stem cells and/or progenitor cells comprising a thrombin-like enzyme, and use of the thrombin like enzyme for activating stem cells and/or progenitor cells.

BACKGROUND ART

Diseases occurring in human are classified roughly as organic diseases and functional diseases. In conventional medicine, medical treatment is mainly used for functional diseases, while surgical treatment and medical treatment are used for organic diseases. However, most of medial treatments are symptomatic treatment, which can not radically cure diseases in the most cases. In addition, surgical treatment is an invasive treatment method, which is practiced by extirpating all or a portion of the injured organ, resulting in partial or complete losses of organ functions. On the other hand, organ transplantation and artificial organs are used as alternative medical methods to surgical treatment. However, a lot of problems exist in organ transplantation, for example, technical problems such as side effects caused by immunosuppressants used to suppress rejection reaction, as well as social problems such as a serious shortage of donors and increased health care costs. In addition, a lot of problems also exist in treatment using artificial organs, which include technical problems such as inability to replace the organic functions and biocompatibility, as well as social problems such as increased health care costs.

Regenerative medicine is currently attracting attention as a new treatment method which solves these problems. Regenerative medicine is termed as a treatment method which aggressively utilizes cells to regenerate construction and restore the function of tissues and organs, for which the functions fall into functional disorder or dysfunction due to illness or accidents. Regenerative medicine is broadly classified into the following three types based on the mode of cell utilization:

(1) a method comprising the steps of collecting embryonic stem cells, placental blood-derived mononuclear cells, blood-derived mononuclear cells or bone marrow-derived mononuclear cells from the donor; inducing the cell proliferation and/or differentiation in vitro culture, and introducing the selected undifferentiated (stem cells and/or progenitor cells) and/or differentiated cells into the patient's body by implantation;

(2) a method comprising the steps of collecting somatic stem cells, blood-derived mononuclear cells or bone marrow-derived mononuclear cells from the said patient, inducing the cell proliferation and/or differentiation in vitro culture, and introducing the selected undifferentiated (stem cells and/or progenitor cells) and/or differentiated cells into the patient himself body by implantation; and

(3) a method comprising the step of stimulating the body of the patient (e.g., by drug administration, physical exercise or physicotherapeutics, etc.) for activating stem cells and/or progenitor cells present in the injured organs or tissues of the patient in situ (it is termed as resident stem cells and/or progenitor cells), and/or blood-derived or bone marrow-derived stem cells and/or progenitor cells of the patient, without inducing proliferation and/or differentiation of stem cells and/or progenitor cells in vitro culture. This method is defined as "self-regeneration".

The current mainstream of regenerative medicine involves methods by which cells are introduced from outside into patient's body by implantation (i.e., the methods of (1) and (2) described above).

Methods in which differentiated cells are introduced from outside of the patient's body are used in the fields of dermatology, ophthalmology and orthopedic surgery which targets tissues or organs (e.g., skin, bone, cartilage, cornea and muscle tissues) constructed from one type or extremely limited types of cells. However, with respect to solid organs (e.g., heart, liver, lungs, kidneys and brain) constructed from multiple types of cells, technology for suitably controlling the behavior of these multiple type of differentiated cells has yet to be adequately established, and not yet reached the practical level.

On the other hand, the following methods are known for introducing undifferentiated cells from outside of the patient's body.

(1) The method of angiogenic therapy involving introduction of stem cells and/or vascular endothelial progenitor cells from outside of the patient's body to promote angiogenesis and form new blood vessels for serious ischemic diseases and cardiovascular diseases (Weissman IL: Translating stem and progenitor cell biology to the clinic: Barriers and opportunities. Science 287:1442-1446,2000).

(2) The method of vasculogenesis utilizing introduction of embryonic stem cells (ES cells) from outside the of patient's body (McCloskey KE et al.: Use of embryonic stem cell-derived endothelial cells as a cell source to generate vessel structures in vitro. Tissue Eng 11:497-505,2005). This method has not reached practical application, since the methods for culturing ES cells, introducing differentiation of the cells and acquiring differentiated cells and so on have not been adequately established.

(3) The method of autologous bone marrow transplantation utilizing bone marrow-derived mononuclear cells isolated from bone marrow of patient himself, and directly introducing the cells into the affected site for formation of new blood vessels (Sato Y et al.: Can a bone marrow cell contribute to organ regeneration? In vivo analysis using transgenic rats with reporter genes. Transplantation Proceedings 37:273-275,2005). This method has problems such as a small number of stem cells is obtainable, physical burden and risk exist in the patient for collection of a large amount of bone marrow by general anesthesia. Moreover, there is also a considerable difficulty in controlling the differentiation of the implanted cells.

In addition, in regenerative medicine using methods involving the introduction of stem cells and/or progenitor cells from outside of the patient's body, a common problem is the risk of the occurrence of complications attributable to excessive regeneration and/or excessive repair by the implanted cells.

In contrast, methods utilizing "self-regeneration" make it possible to recover function by regenerating the injured organs and/or tissues by "activating" stem cells and/or progenitor cells originally present in inside of the patient's body (i.e., resident stem cells and/or progenitor cells, and/or blood-derived or bone marrow-derived stem cells and/or progenitor cells from the patient himself).

Although resident stem cells and/or progenitor cells have conventionally been known to be present in the liver, a regenerative organ, these cells with regenerative ability have recently been reported to also be present in the nervous system (particularly in the central nervous system such as brain), skin, fatty tissue, retina, cornea, skeletal muscle and even in the heart (Garry DJ et al.: Ponce de Leon's fountain stem cells and the regenerating heart. Am J Med Sci 329(4): 190-201, 2005). Currently, it is considered that resident stem cells and/or progenitor cells are present in all organs and tissues (Weissman IL: Translating stem and progenitor cell biology to the clinic: Barriers and opportunities. Science 287:1442-1446,2000; Garry DJ et al.: Ponce de leon's fountain: stem cells and the regenerating heart. Am J Med Sci 329(4):190-201,2005).

Resident stem cells and/or progenitor cells, and/or blood-derived or bone marrow-derived stem cells and/or progenitor cells are known from the literature below to be able to differentiate into different type of tissue cells depending on the different inducing microenvironment condition in which they are present, such as different types of contacting cells, contents of extracellular matrix, cytokines and growth factors containing in the inducing microenvironment condition. For example:

(1) when neural stem cells are cultured in the presence of neurotrophic factor or growth factor, they form single cell-derived colonies referred to as neurospheres (i.e., self-replication), and these neurospheres differentiate into neurons, astrocytes and oligodendrocytes based on different inducing microenvironment condition (i.e., multipotency). In addition, when neurosphere was sub-cultured in vitro, another neurosphere can be formed from single neurosphere-derived cell (Reynolds BA and Weiss S: Generation of neurons and astrocytes from isolated cells of the adult mammalian central nervous system. Science 255:1707-1710,1992);

(2) although neural stem cells only differentiate into gliacytes when neural stem cells obtained from adult rat spinal cord are transplanted into the spinal cord of another adult rat, they differentiate into neurons when transplanted into the hippocampus dentate gyrus (Shihabuddin LS et al.: Adult spinal cord stem cells generate neurons after transplantation in the adult dentate gyrus. The J Neuroscience 20:8727-8735,2000);

(3) when neural stem cells are cultured with vascular endothelial cells, the proliferation of neural stem cells and differentiation into neurons are promoted (Shen Q et al.: Endothelial cells stimulate self-renewal and expand neurogenesis of neural stem cells. Science 304:1338-1440,2004).

On the other hand, for the purpose of regenerative medicine utilizing self-regeneration, the promotion methods for increasing resident stem cells and/or progenitor cells, and/or blood-derived or bone marrow-derived stem cells and/or progenitor cells are known as follows:

(1) when EGF was administered to animals with a model of cerebral ischemia, neural stem cells proliferation was promoted, and about 20% of the lost cells in infarcted area are regenerated (Teramoto T et al.: EGF amplifies the replacement of parvalbumin-expressing striatal interneurons after ischemia. The J Clinical Investigation 111:1125-1132,2003);

(2) when the hyperlipemia therapeutic drug, statin (3-hydroxy-3-methyl-glutaryl coenzyme A (HMG-CoA) reductase inhibitor) was administered to arteriosclerosis patients, the levels of bone marrow-derived hemangioblasts or endothelial progenitor cells increased in the blood (Walter DH et al.: Statin therapy accelerates reendothelialization: A novel effect involving mobilization and incorporation of bone marrow-derived endothelial progenitor cells. Circulation 105:3017-3024,2002);

(3) in addition to the above-mentioned EGF, numerous growth factors such as VEGF, FGF (b-FGF), PDGF, NGF and HGF, cytokines such as G-CSF, GM-CSF, erythropoietin (EPO),and other bioactive substance such as estrogen and lipids, etc. are reported useful for increasing stem cells and/or progenitor cells (Takeyama K, Ohto H: PBSC mobilization. Transfus Apher Sci 31:233-243,2004; Aicher A, Zeiher AM, Dimmeler S: Mobilizing endothelial progenitor cells. Hypertension 45:321-325, 2005). However, there are only two or three of the above-mentioned substances which have been developed into pharmaceuticals such as G-CSF and b-FGF. Moreover, G-CSF has the risk of cancerogenesis, while b-FGF has the risk of side effects such as vascular occlusion during intravenous injection. In addition, in consideration of the diversity of side effects attributable to growth factors targeting numerous types of cells, adequate clinical efficacy has yet to be obtained in clinical studies for VEGF and b-FGF, which were developed as growth factors having a small number of target cell types. Moreover, EPO has side effects including elevation of blood pressure.

In addition, batroxobin, which is known to be a therapeutic drug for acute cerebral infarction and so on, has been reported on the following Chinese Internet web site to affect the expression of CD34 in ischemic tissue during localized ischemia of rat brain.
Author: Cai Zhenli
Title: Expression of CD34 in a rat local cerebral ischemia/reperfusion model and effect of batroxobin on its expression
Related site: Chinese Dissertations Database (CDDB), Index No. Y653774
Media type: Online
Publisher: Wanfang Data
Access Date: July 28, 2005
Address: URL: http://ww.wanfangdata.com.cn/
In this report, the expression of cell surface antigen CD34 was detected in brain tissue specimens containing vessels absent of blood (tissue specimens obtained by removing blood from brain tissue by carrying out coronary perfusion prior to specimen sampling). Here, CD34 is expressed not only in hemangioblasts, hematopoietic stem cells, vascular EPCs and mesenchymal stem cells present in bone marrow, and vascular EPCs and mesenchymal cells (both of which are undifferentiated cells) present in blood, but also in mature vascular endothelial cells (differentiated cells) which compose vessel walls (Ohsawa T et al. ed.: Encyclopedia of Immunology (2nd Edition), p. 327, Tokyo Kagaku Dozin, December 3,2001). Accordingly, in the case of using bone marrow and blood as specimens, although undifferentiated cells (stem cells and progenitor cells) are positive for CD34, in the case of using a specimen consisting of brain tissue containing vessels which are absent of blood, only differentiated cells are CD34 positive. Thus, this report does not make any evaluation whatsoever regarding the action of batroxobin on stem cells and/or progenitor cells.
On the other hand, there is a clinical need for a substance able to be used in regenerative medicine based on a method utilizing self-regeneration which has few or no side effects and is capable to act promptly and moderately depending on the state of advancement and the degree of injured organs and/or tissues to which regenerative medicine is applied (Kinnaird T et al.: Bone-marrow derived cells for enhancing collateral development: mechanism, animal data and initial clinical experiences. Cir Res 95(4):354-363,2004).

DISCLOSURE OF INVENTION

Thus, an object of the present invention is to provide an activating agent of stem cells and/or progenitor cells capable of being used in regenerative medicine, and particularly in regenerative medicine utilizing self-regeneration.
As a result of conducting extensive studies on the relationship between batroxobin, which is a kind of a thrombin-like enzyme, and the activation of stem cells and progenitor cells in order to solve the above-mentioned problems, the inventors of the present invention found that injured organs and tissues can be regenerated by using batroxobin to activate said cells. The present invention was completed on the basis of this finding.
Namely, the present invention relates to an activating agent of stem cells and/or progenitor cells comprising a thrombin-like enzyme and use of the thrombin-like enzyme for activating stem cell and/or progenitor cell.

BRIEF DESCRIPTION OF THE DRAWINGS

FIG. 1 is a bar graph showing the relative numbers of nestin-positive cells in the batroxobin group compared with the model group;
FIG. 2 is a bar graph showing the relative numbers of BrdU-positive cells in the batroxobin group compared with the model group;
FIG. 3 is a bar graph showing the relative numbers of GFAP-positive cells in the batroxobin group compared with the model group;

FIG. 4 is a bar graph showing the relative numbers of BrdU-positive (migrated) cells in the batroxobin group compared with the model group; and

FIG. 5 is a photograph showing the distribution of BrdU-positive cells in the SVZ region in the model group (left) and in the batroxobin group (right). The arrows indicate BrdU-positive cells.

## BEST MODE FOR CARRYING OUT THE INVENTION

The following provides a detailed explanation of the present invention.

The activating agent of stem cells and/or progenitor cells of the present invention is characterized by comprising a thrombin-like enzyme as its active ingredient.

Specific examples of thrombin-like enzyme include batroxobin, ancrod, crotalase and so on (Stocker KF: Snake venom proteins affecting hemostasis and fibrinolysis, in Medical Use of Snake Venom Proteins, Stocker KF, ed., CRC Press, Boston, p130-131;1990). Batroxobin is a most representative of thrombin-like enzyme and is particularly preferable.

Batroxobin is a thrombin-like serine protease derived from the venom of *Bothrops atrox moojeni.* Batroxobin releases only fibrinopeptide A from fibrinogen resulting formation of des A fibrin (i.e., fibrin I) (Aronson DL: Comparison of the actions of thrombin and the thrombin-like venom enzymes Ancrod and Batroxobin. Thrombos Haemostas (stuttg) 36:9-13,1976). In addition, the primary structure of batroxobin has previously been identified as a single chain glycoprotein consisting of 231 amino acids with the molecular weight of about 36,000 Da (Itoh N et al.: Molecular cloning and sequence analysis of cDNA for batroxobin, a thrombin-like snake venom enzyme. J Biol Chem 262:3132-3135, 1987).

Batroxobin is a serine protease in the same manner as thrombin. However, in contrast to batroxobin releases only fibrinopeptide A from fibrinogen resulting formation of des A fibrin, thrombin releases both fibrinopeptides A and fibrinopeptides B from fibrinogen resulting formation of fibrin, thus making it different in that respect. In addition, although batroxobin does not act on blood coagulation factors other than fibrinogen, thrombin differs in that it acts on other blood coagulation factors.

Batroxobin itself is a known substance, and can be prepared according to the method described in Publication of examined Japanese patent application No. 557-10718 (Japanese Patent No. 1118129). Alternatively, it can be easily acquired from Tobishi Pharmaceutical Co., Ltd. (Tokyo, Japan) and its subsidiary, Beijing Tobishi Pharmaceutical Co., Ltd. (Beijing, China).

Ancrod is a thrombin-like serine protease derived from the venom of *Agkistrodon rhodostoma.* Ancrod is a glycoprotein having the molecular weight of about 35,400 Da. As with batroxobin, ancrod releases only fibrinopeptide A from fibrinogen resulting formation of des A fibrin (Stocker KF: Snake venom proteins affecting hemostasis and fibrinolysis, in Medical Use of Snake Venom Proteins, Stocker KF, ed., CRC Press, Boston, p134-135;1990).

Crotalase is a thrombin-like serine protease derived from the venom of *Crotalus adamanteus.* Crotalase is a glycoprotein having the molecular weight of about 32,700 Da. As with batroxobin, crotalase releases only fibrinopeptide A from fibrinogen resulting formation of des A fibrin (Stocker KF: Snake venom proteins affecting hemostasis and fibrinolysis, in Medical Use of Snake Venom Protein, Stocker KF, ed., CRC Press, Boston, p140-141;1990).

The thrombin-like enzymes above-mentioned such as batroxobin, ancrod and crotalase may be naturally occurring substances or genetic recombinant products.

Although the definition of the stem cells and progenitor cells targeted by the present invention have not been completely standardized in the relevant technical field, they are defined in the following manner in the present specification on the basis of the concept that a consensus has been obtained (Weissman IL: Translating stem and progenitor cell biology to the clinic. Barriers and opportunities. Science 287:1442-1446,2000; McKay R: Stem cells hype and hope. Nature 406:361-364,2000).

Stem cells are undifferentiated cells with the self-replication ability and multipotency. Specific examples of stem cells include, somatic stem cells (adult stem cells), hemangioblasts, neural stem cells, hematopoietic stem cells, mesenchymal stem cells and stem cells of other cells (including osteocyte, chondrocyte, myocyte, cardiac myocyte, neuron, tendon cell, adipocyte, pancreocyte, hepatocyte and nephrocyte).

Progenitor cells are undifferentiated cells with the self-replication ability and differentiation potency, but for which type of ultimately differentiated cell is predetermined. Specific examples of progenitor cells include vascular endothelial progenitor cells (EPCs), neuron progenitor cells and hematopoietic progenitor cells.

The self-replication ability, which is one of the criteria for identifying stem cells and progenitor cells, can be evaluated using a test of incorporation of 5-bromodeoxyuridine (BrdU) into DNA (Gould E & Gross CG: Neurogenesis in adult mammals: some progress and problems. The J Neuroscience 22(3): 619-623,2002).

In addition, identification of the type of stem cells and progenitor cells can be carried out on the basis of expression of a characteristic marker on its stem cell and progenitor cell. For example, expression of nestin on neural stem cells (Wiese, C. et al.: Nestin expression - a property of multi-lineage progenitor cells? Cellular and Molecular Life Sciences, 61:2510-2522,2004), or expression of CD34 and CD31 on vascular EPCs (Asahara T et al.: Isolation of putative progenitor endothelial cells for angiogenesis. Science 275: 964-967,1997; Shi Q et al.: Evidence for circulating bone marrow-derived endothelial cells. Blood 92(2):362-367,1998).

Stem cells and progenitor cells present in adults body can be divided into resident cells (i.e., resident stem cells and resident progenitor cells), blood-derived cells (i.e., blood-derived stem cells and blood-derived progenitor cells) and bone marrow-derived cells (i.e., bone marrow-derived stem cells and bone marrow-derived progenitor cells) based on their origin and location.

Resident cells (resident stem cells and/or resident progenitor cells) are defined as cells with self-replication ability and multipotency that are originally present in a specific organ and/or tissue, which contribute to regeneration of that organ and/or tissue in the case said organ and/or tissue is subjected to injury. Specific examples of these resident cells include corneal stem cells, cardiac stem cells, neural stem cells, vascular EPCs and so on.

Blood-derived cells (i.e., blood-derived stem cells and blood-derived progenitor cells) are defined as mononuclear cells with self-replication ability and multipotency that are present in the circulating blood, which contribute to regeneration of an organ and/or tissue by migrating and accumulating into that organ and/or tissue in the case said organ and/or tissue is subjected to injury. The blood in this case includes peripheral blood, placental blood, arterial blood, venous blood, blood sampled from the heart, and blood sampled from the ocular fundus. Specific examples of blood-derived mononuclear cells include vascular EPCs, mesenchymal stem cells and so on.

Bone marrow-derived cells (i.e., bone marrow-derived stem cells and bone marrow-derived progenitor cells) are defined as cells with self-replication ability and multipotency that are originally present in bone marrow, which contribute to regeneration of an organ and/or tissue by migrating through the blood circulation and accumulating into said organ and/or tissue in the case said organ and/or tissue has been subjected to injury, Specific examples of bone marrow-derived cells include vascular EPCs, mesenchymal stem cells and so on.

The present invention is able to activate each of these CD34-expresing resident cells, blood-derived cells and bone marrow-derived cells.

In addition, stem cells and progenitor cells can be divided into activated-state cells (state in which the cells are proliferating, differentiating or migrating) and inactive-state cells (state in which cells are not proliferating, differentiating or migrating). But the activating agent of the present invention is able to activate stem cells and progenitor cells in both activated-state and inactive-state.

The activating agent of the present invention activates CD34 expressing stem cells and/or progenitor cells. Here, "activate" is defined as the three actions indicated below.

(1) Promotion of proliferation of stem cells and/or progenitor cells.
(2) Promotion of migration of stem cells and/or progenitor cells. Migration is defined as stem cells and/or progenitor cells being mobilized from bone marrow, organ and/or tissue into the circulating blood; accumulated in the injured organ and/or tissue from the circulating blood; or moved inside of the organ and/or tissue.
(3) Promotion of differentiation of stem cells and/or progenitor cells.

Targeted stem cells and/or progenitor cells by the activating agent of the present invention are stem cells and/or progenitor cells applied to a treatable disease by regenerative medicine (including regenerative medicine in which cells are introduced from outside of the patient's body and regenerative medicine utilizing self-regeneration).

Thus, the activating agent of the present invention can be used not only for stem cells and/or progenitor cells used in regenerative medicine utilizing self-regeneration (i.e., cells that are originally possessed by a patient, and are not subjected to the inducement of proliferation and/or differentiation in vitro culture), but also for stem cells and/or progenitor cells used in regenerative medicine in which cells are introduced from outside of the patient's body (i.e., cells introduced from outside of the patient's body). The activating agent of the present invention can be preferably used for CD34 expressing stem cells and/or progenitor cells used in regenerative medicine using self-regeneration.

There are no particular limitations on the cause, type or degree of diseases (injured organs or injured tissues) to which regenerative medicine is applied. The followings are the specific examples of these diseases.

Skin Diseases

Wound, burn, radiation injury, frostbite, ultraviolet injury, electrical shock, trauma, skin ulcer, bedsore, contact dermatitis, bullous dermatitis, atopic dermatitis, xeroderma, diabetic skin ulcers, autosensitization dermatitis, erythroderma, exfoliative dermatitis, epidermolysis bullosa, photodermatosis, chronic pigmented purpura (Schamberg's disease), injury of oral mucosa, stomatitis, peristomal dermatitis, skin aging symptoms, alopecia, paronychia, unguis incarnatus, gastrointestinal mucosa erosion, digestive ulcer, corneal erosion, corneal ulcer, caries, pulpitis, marginal periodontitis, allergic rhinitis, pollenosis, vernal conjunctivitis, hemorrhoid, gastrointestinal mucosa injury, gastrointestinal mucosa burn, bronchial asthma, glossitis, recurrent aphtha, intraoral aphtha, bad breath, oral abnormal sensation, dental infection, oral mucosa bites, tongue bites, oral mucosa burn, and oral mucosa ulcer.

Cranial Nerve Diseases

Stroke, Alzheimer's disease, Parkinson's disease, various central nervous system diseases (e.g., myelitis), various peripheral nerve diseases (e.g., multiple peripheral neuropathy), myelopathy and various types of encephalitis.

Cardiovascular Diseases

Myocardial infarction, angina, unstable angina, various types of myocarditis (e.g., viral myocarditis), acute cardiac insufficiency, chronic cardiac insufficiency, atherosclerosis, hypertension, rheumatoid heart disease, arrhythmia, valvular heart disease, infectious endocarditis, pericarditis, percutaneous coronary artery intervention, and restenosis and reobstruction following PTCA.

Gastrointestinal Diseases

Esophagitis, acute gastritis, chronic gastritis, gastric ulcer, duodenal ulcer, various types of colitis (e.g., ulcerative colitis), intestinal tuberculosis, viral hepatitis, alcoholic hepatitis, drug-induced hepatitis, fatty liver, cirrhosis, pancreatitis, and organ disorders caused by surgery for various types of gastrointestinal cancers (e.g., liver cancer, colon cancer and stomach cancer).

Respiratory Diseases

Various types of bronchitis (e.g., bacterial bronchitis), infectious pneumonia, inhalation pneumonia, pulmonary embolism, pneumothorax and pulmonary insufficiency.

Urological Diseases

Cystitis, various types of nephritis (e.g., chronic nephritic syndrome, primary glomerular disease), adrenalitis, urethritis, bacterial and non-bacterial prostatitis, hypertensive nephropathy, diabetic nephropathy and renal insufficiency.

Locomotor Diseases

Various types of arthritis (e.g., rheumatoid arthritis), muscular atrophy, bone defects following craniotomy during neurosurgery, bone defects following tumorectomy during orthopedic surgery, bone defects following fractures, bone defects caused by periodontosis in the field of dentistry, chondritis, cartilage defects of various joints, tendon injuries caused by various injuries (e.g., trauma or distortion).

Vascular Diseases

Various types of arterial diseases (e.g., arteriosclerosis obliterans (ASO)), Buerger's disease (thromboarteritis obliterans (TAO)), various types of venous diseases (e.g., thrombophlebitis), thrombosis, circulatory disorders, deep vein thrombosis (DVT), various types of diseases accompanying peripheral circulatory disorders (e.g., sudden deafness and vibration disease), and restenosis and reobstruction following angioplasty.

Endocrine Diseases

Diabetes and its complications (e.g., diabetic peripheral neuropathy, diabetic foot and diabetic ulcer), hyperlipemia, thyroiditis and obesity.

Ophthalmological Diseases

Various types of keratitis (e.g., keratitis caused by alkaline or acidic compounds and traumatic keratitis), and diabetic retinitis.

In addition to the diseases listed above, stem cells and/or progenitor cells used in regenerative medicine for diseases that are able to be treated by activation of stem cells and/or progenitor cells (i.e., regeneration of an injured organ and/or injured tissue) are also targets of the activating agent of the present invention. Furthermore, there are no particular limitations on the advanced state of the disease to which regenerative medicine is applied.

In addition, the activating agent of the present invention is able to act promptly and moderately depending on the advanced state and the degree of the injured organ and/or injured tissue to which regenerative medicine is applied.

The activating agent of the present invention can be preferably applied to stem cells and/or progenitor cells present in patients with skin diseases, locomotor diseases, endocrine diseases, respiratory diseases, urological diseases, gastrointestinal diseases, ophthalmological diseases, cranial nerve diseases, cardiovascular diseases and vascular diseases, and particularly preferably applied to stem cells and/or progenitor cells present in patients with cranial nerve diseases, cardiovascular diseases, skin diseases and vascular diseases.

Furthermore, the activating agent of the present invention has few or no side effects.

Moreover, the activating agent of the present invention is able to demonstrate activation effects sustainably.

The activating agent of the present invention may be comprised of a thrombin-like enzyme alone (e.g., batroxobin' alone) or one more thrombin like enzymes, and may be comprised of the combination of the thrombin-like enzyme with one or more any active substance other than the thrombin-like enzymes.

Examples of other active substances include growth factors such as VEGF, EGF, FGF, PDGF, NGF and HGF, cytokines such as G-CSF, GM-CSF and EPO, and estrogen, lipids, statin (coenzyme A reductase inhibitor), anti-growth factor antibody, growth factor inhibitor, anti-growth factor inhibitor antibody, and anti-cytokine antibody (Takeyama K, Ohto H: PBSC mobilization. Transfus Apher Sci 31:233-243, 2004; Aicher A, Zeiher AM, Dimmeler S: Mobilizing endothelial progenitor cells. Hypertension 45:321-325,2005).

Any formulation in Japanese Pharmacopoeia General Rules for Preparations can be applied to the formulation of the activating agent of the present invention. Examples of the formulation of the activating agent presently disclosed include injections for direct application inside the body (including suspensions and emulsions); ointments (including fatty ointments, emulsion ointments (creams), water-soluble ointments and the like), inhalants, liquids (including ophthalmic solutions, collunarium and the like), suppositories, patches, poultices, lotions and other external formulations; and internal formulations including tablets (including sugar-, film- and gelatin-coated), liquids, capsules, granules, powders (including grains), pills, syrups, troches and the like. These formulations can be prepared by the methods described in the Japanese Pharmacopoeia General Rules for Preparations.

In addition, the activating agent of the present invention may also include pharmacologically acceptable solid or liquid carriers or interventional therapy bases. Examples of pharmacologically acceptable solid or liquid carriers include solvents, stabilizers, preservatives, solubilizing agents, emulsifiers, suspending agents, buffering agents, isotonizing agents, coloring agents, bases, thickeners, excipients, lubricants, binding agents, disintegrating agents, coating agents, corrigents and the like.

Specific examples include water, lactose, sucrose, fructose, glucose, mannitol, sorbitol and other sugars and sugar alcohols, crystalline cellulose, methylcellulose, ethylcellulose, hydroxypropylcellulose, low substituted hydroxypropylcellulose, hydroxypropylmethylcellulose, hydroxypropylmethylcellulose phthalate, hydroxypropylmethylcellulose acetate succinate, carmellose, carmellose calcium, carmellose sodium, croscarmellose sodium, carboxymethylethylcellulose, cellulose acetate phthalate and other celluloses and related derivatives, corn starch, wheat starch, rice starch, potato starch, dextrin, pregelatinized starch, partly pregelatinized starch, hydroxypropyl starch, sodium carboxymethyl starch, cyclodextrin, pullulan and other starches and related derivatives, agar, sodium alginate, acacia, gelatin, collagen, shellac, tragacanth, xanthan gum and other natural polymers (seaweeds, plant mucilage, proteins and the like), polyvinylpyrrolidone, aminoalkyl methacrylate copolymer, methacrylic acid copolymer, carboxyvinyl polymer, polyvinyl alcohol, dimethylpolysiloxane and other synthetic polymers, olive oil, cacao butter, carnauba wax, beef tallow, hydrogenated oil, soybean oil, sesame oil, camellia oil, paraffin, liquid paraffin, yellow beeswax, white petrolatum, coconut oil, microcrystalline wax and other oils and fats, stearic acid, aluminum stearate, calcium stearate, magnesium stearate, triethyl citrate, triacetine, medium chain fatty acid triglyceride, hard fat, isopropyl myristate and other fatty acids and derivatives thereof, glycerin, stearyl alcohol, cetanol, propylene glycol, macrogol and other alcohols and polyvalent alcohols, zinc oxide, dibasic calcium phosphate, precipitated calcium carbonate, synthetic aluminum silicate, silicon dioxide anhydride, kaolin, dried aluminum hydroxide gel, synthetic hydrotalcite, titanium oxide, talc, bentonite, magnesium aluminometasilicate, aluminum potassium sulfate, bismuth subgallate, bismuth subsalicylate, calcium lactate, sodium bicarbonate and other inorganic substances and metal salt compounds, sucrose esters of fatty acid, polyoxyl stearate, polyoxyethylene hydrogenated castor oil, polyoxyethylene polyoxypropylene glycol, sorbitan sesquioleate, sorbitan trioleate, sorbitan monostearate, sorbitan monopalmitate, sorbitan monolaurate, polysorbate, glyceryl monostearate, sodium lauryl sulfate, lauromacrogol and other surfactants, dyes, perfumes and the like.

Examples of interventional therapy bases include stents, artificial blood vessels, catheter, balloon and the like.

For example, the product of the activating agent of the present invention can contain the following ingredients and each amount in total volume of 1ml, as shown in Table 1.

Table 1. The compositions of batroxobin product

| Ingredient | Content |
|---|---|
| Batroxobin (active ingredient) | 10BU |
| Chlorobutanol (preservative) | 3mg |
| Gelatin hydrolvzate (stabilizer) | 0.1mg |
| Sodium chloride (isotonic agent) | 9mg |
| Hydrochloric acid (pH regulator) | q.s. |
| Distilled water for injection | up to 1ml |
| Total volume | 1ml |

The administered dose of the activating agent varies depending on the patient's weight, disease's property and condition, but is for example 0.1 to 50 batroxobin units (BU) of batroxobin once per day, preferably 1 to 20 BU of batroxobin once every other day in the case of an adult.

The batroxobin unit described herein is a unit representing an enzyme activity of batroxobin and such an activity that the coagulation of plasma is taken place in $19.0 \pm 0.2$ seconds when 0.1 ml of a batroxobin solution is added to 0.3 ml of standard human plasma containing citric acid at a temperature of 37°C is defined as 2 BU.

The activating agent can be administered by intravenous drip, intravenous injection, intraarterial injection, intramuscular injection, subcutaneous injection, intradermal injection, intracardiac injection, intraperitoneal injection, intrathecal injection, rectal administration, sublingual administration, nasal administration, percutaneous administration, inhalation or local administration into the injured organ and/or tissue after suitably diluting the batroxobin with physiological saline. In general, the activating agent of the present invention is preferably infused over the course of 1 hour or more after diluting with 100 ml or more of physiological saline.

The acute toxicity ($LD_{50}$ (BU/kg)) of batroxobin in mice, rats, rabbits and dogs is as shown in Table 2 below. Acute toxicity studies were conducted by intravenous administration of batroxobin according to methods described in the literature (Ozaki M et al.: Toxicity of the defibrase, Defibrinogen Batroxobin (First Report), Pharmacometrics 25:339-346,1983).

Table 2. Acute Toxicity of Batroxobin (i.v.)

| Animal Species | $LD_{50}$ Value (BU/kg) |
|---|---|
| Mouse (ddy) | 192~210 |
| Rat (Wistar) | 105~110 |
| Rabbit (NW) | >300 |
| Dog (mongrel) | 190~208 |

The activating agent is applicable to the animal having stem cell and/or progenitor cell. Specific examples of the animal include human, monkey, dog, pig, cat, rabbit, rat and mouse. Among them, human is preferable.

Although the followings provide a detailed explanation of the present invention by indicating Examples thereof, the present invention is not limited to these Examples.

Example 1

Effect of Batroxobin on Activation of Cord Blood-Derived CD34-Positive Mononuclear Cells

In the present example, the effect of batroxobin on activation of cord blood-derived CD34-positive mononuclear cells was evaluated in vitro.

Furthermore, those cells present in cord blood corresponding to CD34 positive mononuclear cells are known to consist of vascular EPCs and mesenchymal stem cells (Murohara T et al.: Transplanted cord blood-derived endothelial precursor cells augment postnatal neovascularization. J Clin Invest 105:1527-1536,2000). Thus, the human cord blood-derived CD34-positive mononuclear cells evaluated in the present example can be said to be vascular EPCs and mesenchymal stem,cells.

Experimental Method:

(1) Collection of Human Cord Blood Mononuclear Cells

Normal pregnancies with full term birth were selected and 40~60 ml of cord blood was collected from the umbilical cord and placenta using heparin (20~30 U/ml) as a anticoagulant. The collected cord blood was mixed in a ratio of 5:1 with 6% hydroethylstarch (Becton Dickinson, NJ, USA) and allowed to stand incubated for 30 minutes followed by removal of the precipitated red blood cells. Next, the liquid of the upper layer was collected and suspended at a ratio of 2:1 in lymphocyte separation liquid (relative density: 1.077, Chinese Academy of Medical Sciences, Tianjin, China), and it was centrifuged for 25 minutes under conditions of 460xg. The mononuclear cells in the intermediate cell layer were collected and washed twice with phosphate-buffered saline (PBS) prior to use in the experiment.

(2) Separation and Identification of CD34-Positive Mononuclear Cells

The human cord blood-derived mononuclear cells obtained in Experimental Method (1) above were suspended in PBS to prepare a cell suspension with concentration of $2 \times 10^6$ cells/ml. CD34-positive mononuclear cells were separated from the human cord blood-derived mononuclear cells using the CD34-Positive Cell Monoclonal Immunomagnetic Bead Separation Kit (MACS, Milteny Biotech, Bergisch Gladbach, Germany) in accordance with the instructions provided with the kit. The quality of resulting CD34-positive mononuclear cells was evaluated by flow cytometry (Becton Dickinson FACS Vantage, Becton Dickinson, NJ, USA) using FITC-labeled anti-CD34 antibody (Becton Dickinson, NJ, USA). The purity of obtained CD34-positive cells was 98.5% or more.

(3) Cell Migration Test

This test was carried out using a modified Boyden's chamber which consists of a 24-well culture plate (Corning, NY, USA) and a 5 μm transwell (Corning, NY, USA). The modified Boyden's chamber is divided into two compartments by a polycarbonate membrane filter with 5 μm (diameter) pore size. The upper compartment is the space above the filter, and the lower compartment is the gap between the filter and wells.

Six hundred μl each of various concentrations of batroxobin (0 (control group), 0.1 and 0.2 BU/ml) prepared using 0.25% BSA IMDM (Gibco, Los Angeles, USA) were added to the lower compartment of the Boyden's chamber. Next, 100 μl of cell suspension with a concentration of $1 \times 10^5$ cells/ml of the human cord blood-derived CD34-positive mononuclear cells in IMDM medium were added the upper compartment of each Boyden's chamber. This system was incubated in a humidified incubator at 37°C under an atmosphere of 5% $CO_2$/95% air for 6 hours in the stationary state. After incubation, the cells in the lower compartment which moved through the filter from the upper compartment were defined as migrated cells. The migrated cells were collected and the number was counted using flow cytometry.

On the basis of the resulting number of migrated cells, the cell migration rates were calculated for the 0.1 BU/ml batroxobin group and the 0.2 BU/ml batroxobin group based on a value of 100% for the number of migrated cells of the control group. The cell migration rate was calculated as the mean ± standard deviation (SD) (%) based on data obtained in two experiments carried out in triplicate under same conditions.

Results and Discussion

When the migration rate of CD34-positive cells in the control group (0 BU/ml) was defined as 100%, the migration rate in the 0.1 BU/ml batroxobin group was 149.37±6.82%, and.in the 0.2 BU/ml batroxobin group was 254.26±17.44%. Namely, both of the 0.1 and 0.2 BU/ml of batroxobin group shown a significantly higher migration rate than the control group.

Those results indicate that batroxobin activated human cord blood-derived CD34-positive mononuclear cells (i.e., vascular EPCs and mesenchymal stem cells) significantly. The activating effect of batroxobin is considered to be on promoting migration of vascular EPCs and mesenchymal stem cells.

Furthermore, although cord blood was used as the source of the CD34-positive mononuclear cells in the present example, CD34-posiive mononuclear cells are also known to be present in adult peripheral blood, adult bone marrow and fetal bone marrow (Michejda M: Which stem cells should be used for transplantation? Fetal Diagn Ther 19:2-8,2004). Thus, batroxobin is also able to activate CD34-positive mononuclear cells present in blood and bone marrow other than cord blood such as adult peripheral blood, adult bone marrow, fetal bone marrow and other origin of blood and so on.

Example 2

Activation of CD34-Positive Cells, CD34-Positive/CD31-Positive Cells and VE Cadherin-Positive Cells in Peripheral Blood of Lower Limb Deep Vein Thrombosis Patients, and Effects of Batroxobin on Functional Recovery of Damaged Tissue in Lower Limb Deep Vein Thrombosis Patients

In the present example, batroxobin was administered to patients with lower limb deep vein thrombosis (lower limb DVT), a kind of vascular disease, to evaluate the effects of batroxobin on activation of CD34-positive cells, CD34-positive/CD31-positive cells and VE cadherin-positive cells in the peripheral blood, and the effects of batroxobin on regeneration of vessels damaged by thrombi.

Furthermore, those cells present in peripheral blood which are considered to be CD34-positive mononuclear cells are known to be vascular EPCs and mesenchymal stem cells (Zhao Y et al.: A human peripheral blood monocyte-derived subset acts as pluripotent stem cells. Proc Natl Acad Sci USA 100: 2426-2431, 2003). Thus, the CD34-positive mononuclear cells in the peripheral blood evaluated in the present example can be said to be vascular EPCs and mesenchymal stem cells.

In addition, endothelial progenitor cells are known to express CD34 (Michejda M: Which stem cells should be used for transplantation? Fetal Diagn Ther 19:2-8,2004; Fadini GP et al.: Circulating endothelial progenitor cells are reduced in peripheral vascular complications of tape 2 diabetes mellitus. J American College of Cardiology 45:1449-1457, 2005; Kuwana M et al.: Human circulating CD14+ monocytes as a source of progenitors that exhibit mesenchymal cell differentiation. J Leukoc Biol 74:833-845,2003) and CD31 (Asahara T et al.: Isolation of putative progenitor endothelial cells for angiogenesis. Science 275:964-967,1997; Hristov M, Weber C: Endothelial progenitor cells: characterization, pathophysiology, and possible clinical relevance. J Cell Mol Med 8:498-508,2004) as markers. Moreover, vascular EPCs, in which differentiation has progressed and are in a state of being able to adhere to blood vessels, are also known to further express VE cadherin (Hristov M, Weber C: Endothelial progenitor cells: characterization, pathophysiology, and possible clinical relevance. J Cell Mol Med 8:498-508,2004). Thus, the CD34-positive/CD31-positive mononuclear cells and VE cadherin-positive mononuclear cells in peripheral blood evaluated in the present example can be said to be vascular EPCs.

Experimental Method

(1) Test Subjects

The subjects consisted of 11 male and 4 female lower limb DVT patients with age of 37 to 80 years old(average age: 64 years old). The purpose of the clinical test was explained to all subjects and their consent was obtained before conducting the clinical test.

(2) Administration Method

Batroxobin was administered to the patients by intravenous drip for 14 consecutive days at 10 BU/body/day initially (on the day of admission in the case a patient was admitted in the morning, or on the day after admission in the case a patient was admitted in the afternoon), and at 5 BU/body/day thereafter.

(3) Blood Sampling

Five ml of peripheral blood was collected from the median cubital vein of the forearm of each patient using low molecular weight heparin anticoagulant before administration (before initial administration) and on day 7 and 14 after administration. The collected blood was immediately stored at 4°C and measured within 6 hours after collection. Blood samples were collected from all 15 lower limb DVT patients before administration and on day 7 after administration, and from 10 of the 15 lower limb DVT patients on day 14 after administration.

(4) Separation of Peripheral Blood Mononuclear Cells

Two point five ml of lymphocyte separation liquid (Ficoll, Chinese Academy of Medical Sciences, Tianjin, China) was added to 5 ml of the blood obtained in Experimental Method (3) above to prepare a suspension. After centrifuging the suspension at the condition of 460xg, the intermediate cell layer was recovered as the peripheral blood-derived mononuclear cells. The total number of the resulting mononuclear cells was about $5\sim6\times10^6$ cells per patient. The resulting mononuclear cells were suspended in phosphate-buffered saline (PBS) to prepare cell suspension with a concentration of $2\times10^6$ cells/ml.

(5) Isolation of CD34-Positive Mononuclear Cells

CD34-positive mononuclear cells were isolated by immunofluorescent stain using flow cytometry. Twenty μl of phycoerythrin-labeled anti-CD34 antibody (Becton Dickinson, NJ, USA) was added to 100 μl of the mononuclear cell suspension obtained in Experimental Method (4) above and allowed to incubate for 30 minutes at 4°C and under the dark condition. Next, CD34-positive mononuclear cells were isolated and counted by flow cytometry. The ratio (%) of CD34-positive mononuclear cells among the mononuclear cells was calculated according to the following formula.

$$\text{CD34-positive mononuclear cells (\%)} = (\text{No. of CD34-positive mononuclear cells/total No. of mononuclear cells}) \times 100$$

(6) Isolation of CD34-Posiive/CD31-Positive Mononuclear Cells

CD34-positive/CD31-positive mononuclear cells were isolated by double-immunofluorescent stain using flow cytometry. Twenty μl of phycoerythrin-labeled anti-CD34 antibody (Becton Dickinson, NJ, USA) and 20 μl of FITC-labeled anti-CD31 antibody (Becton Dickinson, NJ, USA) were added to 100 μl of the mononuclear cell suspensions obtained in Experimental Method (4) above and allowed to incubate for 30 minutes at 4°C and under the dark condition. Next, the CD34-positive/CD31-positive mononuclear cells were isolated and counted by flow cytometry. The ratio (%) of CD34-positive/ CD31-positive mononuclear cells among the mononuclear cells was calculated according to the following formula.

$$\text{CD34-positive/CD31-positive mononuclear cells(\%)} = (\text{No. of CD34-positive/CD31-positive mononuclear cells/total No. of mononuclear cells}) \times 100$$

(7) Isolation of VE Cadherin-Positive Mononuclear Cells

VE Cadherin-positive mononuclear cells were isolated by immunofluorescent stain using flow cytometry. Twenty μl of FITC-labeled anti-VE cadherin antibody (Becton Dickinson, NJ, USA) were added to 100 μl of the mononuclear cell suspensions obtained in Experimental Method (4) above and allowed to incubate for 30 minutes at 4°C and under the dark condition. Next, VE cadherin-positive mononuclear cells were isolated and counted by flow cytometry. The ratio (%) of VE cadherin-positive mononuclear cells among the mononuclear cells was calculated according to the following formula.

$$\text{VE cadherin-positive mononuclear cells(\%)} = (\text{No. of VE cadherin-positive mononuclear cells/total No. of mononuclear cells}) \times 100$$

(8) Measurement of Girth of Above-Knee and Below-Knee

Lower limb edema is a typical and objective symptom of lower limb DVT. Therefore, the degree of lower limb edema is evaluated based on a lower limb girth in 20 cm of above-knee and in 15 cm of below-knee measured before and after administration of batroxobin in the 15 patients.

Results and Discussion

(1) Activation of CD34-Positive Mononuclear Cells

Table 3. The changes of CD34-positive mononuclear cells in the peripheral blood of lower limb DVT patients (mean±SD, %)

| | No. Patients | CD34-Positive Mononuclear Cells |
|---|---|---|
| Before administration | 15 | 0.39±0.43 |
| Day 7 after administration | 15 | 0.71±0.51* |
| Day 14 after administration | 10 | 1.10±0.66** |
| *Indicates P<0.05; and **indicates P<0.01 as compared with the before administration using the paired-sample non-parameter test. | | |

As a result of administration of batroxobin, the percentage of CD34-positive mononuclear cells in the peripheral blood increased significantly as compared with the value of before administration (Table 3).

This result indicates that batroxobin activated CD34-positive mononuclear cells (i.e., vascular EPCs and mesenchymal stem cells) of the peripheral blood in the lower limb DVT patients. The activating effect of batroxobin is considered to be on promoting proliferation and migration of the CD34-positive mononuclear cells.

(2) Activation of CD34-Positive/CD31-Positive Mononuclear Cells

Table 4. The changes of CD34-positive/CD31-positive mononuclear cells in the peripheral blood of lower limb DVT patients (mean±SD, %)

| | No. Patients | CD34-Positive/CD31-Positive Mononuclear Cells |
|---|---|---|
| Before administration | 15 | 0.28±0.30 |
| Day 7 after administration | 15 | 0.69±0.50** |
| Day 14 after administration | 10 | 1.07±0.66** |
| *Indicates P<0.05; and **indicates P<0.01 as compared with the before administration using the paired-sample non-parameter test. | | |

As a result of administration of batroxobin, the percentage of CD34-positive/CD31-positive mononuclear cells in the peripheral blood increased significantly as compared with the value of before administration (Table 4).

This result indicates that batroxobin activated CD34-positive/CD31-positive mononuclear cells (i.e., vascular EPCs and mesenchymal stem cells) of the peripheral blood in lower limb DVT patients. The activating effect of batroxobin is considered to be on promoting proliferation, migration and differentiation of CD34-positive/CD31-positive mononuclear cells.

(3) Activation of VE Cadherin-Positive Mononuclear Cells

Table 5. The changes of VE cadherin-positive mononuclear cells in the peripheral blood of lower limb DVT patients (mean±SD, %)

| | No. Patients | VE Cadherin-Positive Mononuclear Cells |
|---|---|---|
| Before administration | 15 | 0.44±0.75 |
| Day 7 after administration | 15 | 1.25±1.39* |
| Day 14 after administration | 10 | 1.61±2.60 |
| *Indicates P<0.05 as compared with the before administration using the paired-sample non-parameter test. | | |

As a result of administration of batroxobin, the percentage of VE cadherin-positive mononuclear cells in the peripheral blood increased significantly as compared with the value of before administration (Table 5).

This result indicates that batroxobin activated VE cadherin-positive mononuclear cells (i.e., vascular EPCs) of the peripheral blood in lower limb DVT patients. The activating effect of batroxobin is considered to be on promoting proliferation, migration and differentiation of VE cadherin-positive mononuclear cells.

(4) Improvement of the edema symptoms of lower limb DVT

Table 6. The changes of girth of above-knee and below-knee in lower limb DVT patients (mean±SD, cm)

|  | No. of Patients | Girth of Above-Knee | Girth of Below-Knee |
|---|---|---|---|
| Before administration | 15 | 55.7±6.2 | 38.1±4.2 |
| Day 14 after administration | 15 | 51.8±6.0** | 35.0±2.7## |
| **indicates P<0.01 as compared with the before administration using the Wilcoxon's test, ## indicates P<0.01 as compared with the before administration using the paired-sample t-test. | | | |

The degree of lower limb edema is evaluated based on a lower limb girth in 20 cm of above-knee and in 15 cm of below-knee. The girth of above-knee and below-knee decreased significantly by administration of batroxobin (Table 6).

This result indicates that stem cells and/or progenitor cells are activated by administration of batroxobin which is considered to be on promoting proliferation, migration and differentiation of the stem cells and/or progenitor cells, resulting in damaged vessels are regenerated and edema symptoms are improved in the lower limb DVT patients.

Furthermore, when plasma fibrinogen level is analyzed as an indicator of the pharmacological action of batroxobin, fibrinogen level is found to decrease significantly on day 7 and 14 after administration of batroxobin (data not shown). This indicates that the administered batroxobin acts very well in vivo.

In addition, although the parameters of APTT, PT and TT, which serve as indicators of side effects (such as bleeding) manifesting during administration of preparations acting on the blood coagulation and fibrinolysis systems (including batroxobin) were analyzed on before administration, day 7 and 14 of after administration of batroxobin, there were no significant differences between before and after administration of batroxobin (data not shown). These results indicate that there is no occurrence of side effects by administration of batroxobin.

Example 3

Effects of Batroxobin on Activation of Neural Stem Cells and/or Neural Progenitor Cells, and Recovery of Neural Function in a Cerebral Ischemia/Reperfusion Injury Model

In this example, the effects of batroxobin on activation of neural stem cells and/or neural progenitor cells, and recovery of neural function were evaluated using a rat cerebral ischemia/reperfusion injury model.

Experimental Method:

(1) Animals

Male Sprague-Dawley rats with age of 12 weeks and weight of 250~280 g (Shanghai Animal Center, Chinese Academy of Medical Sciences, Shanghai, China) were used in the experiment after acclimating for 1 week.

(2) Establishment of Cerebral Ischemia/Reperfusion Injury Model

A middle cerebral artery occlusion model was first established in compliance with the method of Longa EZ et al. (Longa EZ et al.: Reversible middle cerebral artery occlusion without craniectomy in rats. Stroke 20:84-91,1989). More specifically, 0.36g/kg of 10% chloral hydrate (Shencheng Chemical Co., Shanghai, China) was administered intraperitoneally to rats which had been fasted for 12 hours (although given free access to water) to anesthetize the rats. Next, a midline incision was made in the neck to expose the right common carotid artery. Then, the internal carotid artery and external carotid artery were separated, the posterior carotid artery, which is a branch of the external carotid artery, and the superior thyroid artery were coagulated and severed, the external carotid artery was ligated at the branch of the lingual artery and maxillary artery, a small puncture was formed in the external carotid artery, 4-0 Nylon suture was passed through the common carotid artery from the puncture, and gradually inserted into the internal carotid artery until there was resistance. The length of the Nylon suture was about 18~20 mm from the branch of the common carotid artery. As a result of occluding the middle cerebral artery with Nylon suture, an ischemic state was applied for 2 hours to create a middle cerebral artery occlusion model.

A cerebral ischemia/reperfusion injury model was established by removing the Nylon suture from the middle cerebral artery of the occlusion model and following reperfusion of blood.

(3) <u>Administration of Batroxobin to Model Rats</u>

The rats were divided into three groups and respectively treated in the manner described below.

1) Sham-operation group (75 rats): The above-mentioned procedure (2) was performed in the rats just for separation of the internal carotid artery and external carotid artery, subsequent occlusion of the middle cerebral artery and reperfusion procedures were not performed. The rats in this group were administered same volume of physiological saline alternative of batroxobin intraperitoneally on the same times of administration as indicated in batroxobin group.

2) Model group (75 rats): These animals were used to establish the cerebral ischemia/reperfusion injury model. The model rats in this group were administered same volume of physiological saline alternative of batroxobin intraperitoneally on the same times of administration as indicated in batroxobin group.

3) Batroxobin group (75 rats): These animals were used to establish the cerebral ischemia/reperfusion injury model, after which a batroxobin preparation diluted with physiological saline was administered intraperitoneally at a dose of 20BU/kg/10ml at 30 minutes after the onset of ischemia, and ischemia was maintained for further one hour and 30 minutes, followed by reperfusing by removing the Nylon suture. The batroxobin was also administered intraperitoneally at a dose of 20BU/kg/10ml on day 2, 4, 6 and 8 after the ischemia/reperfusion injury model established.

(4) <u>Evaluation of Neural Function Score</u>

Neural function scores were evaluated once a day after the ischemia/reperfusion injury model established in the 15 rats per group. The scores of neural function disorders were evaluated using the following criteria in compliance with the method of Longa EZ (Longa EZ et al: Reversible middle cerebral artery occlusion without craniectomy in rats. Stroke 20:84-91,1989). The results were shown as median.

0: Normal
1: Limb bending on opposite side of infarction focus
2: Limb on opposite side of infarction focus becomes asthenic state when tail is pulled backward
3: Turning toward opposite side of infarction focus when tail is pulled
4: Spontaneous turning towards opposite side of infarction focus
5: Loss of spontaneous movement

(5) <u>Analysis of Nestin-Positive Cells</u>

Ten rats of each group were autopsied on day 7, 14 and 21 after the model established, respectively (total animals are 30 rats from each group). After preparation of tissue specimens, the neural stem cells were counted under a microscope.

More specifically, rat frozen brain sections were prepared on day 7, 14 and 21 after the model established, and the presence of nestin-positive cells in the subventricular zone (SVZ) was measured by immunohistochemical stain using the marker of nestin expressed on the neural stem cells. The presence of nestin-positive cells was analyzed using mouse anti-rat nestin monoclonal antibody (Chemicon, Temecula, USA).

(6) <u>Analysis of BrdU-Positive Cells and BrdU-Positive/Nestin-Positive Cells</u>

On day 5, 6, 12, 13, 19 and 20 after the model established, 50mg/kg of 5-bromodeoxyuridine (BrdU, CalBiochem, San Diego, USA) was injected intraperitoneally into 30 rats in each group. Because BrdU is a thymidine analog, it can incorporate into the DNA of cells during the course of cell proliferation (DNA synthesis). Thus, cell proliferation can be analyzed by detecting the amount of intracellular BrdU. The rats were autopsied on day 7, 14 and 21 after the model established for preparation of frozen brain sections. The presence of BrdU-positive cells by immunochemical stain, and the presence of BrdU-positive/nestin-positive cells by double-immunofluorescent stain in the SVZ region were analyzed.

The presence of BrdU-positive cells was analyzed using mouse anti-rat BrdU monoclonal antibody (Oncogene, Westhaver, USA).

The presence of BrdU-positive/nestin-positive cells was analyzed by double-immunofluorescent stain using a combination of primary antibody of goat anti-rat BrdU polyclonal antibody (Biodesign, Saco, USA) and secondary antibody of FITC-labeled rabbit anti-goat antibody (Pierce, Rockford, USA), and a combination of primary antibody of mouse anti-rat nestin monoclonal antibody and secondary antibody of Rho-labeled rabbit anti-mouse antibody (Pierce, Rockford, USA).

(7) Analysis of NeuN-Positive Cells, GFAP-Positive Cells, and-BrdU-Positive/GFAP Positive Cells

On day 5, 6, 12, 13, 19 and 20 after the model established, 50mg/kg BrdU was injected intraperitoneally into 30 rats in each group. The rats were autopsied on day 7, 14 and 21 after the model established for preparation of frozen brain sections. The presences of NeuN-positive cells by immunochemical stain, glial fibrillary acidic protein (GFAP)-positive cells by immunochemical stain, and BrdU-Positive/GFAP Positive Cells by double-immunofluorescent stain in the SVZ region were analyzed. NeuN is a marker expressed on the immature neurons which are differentiated from neural stem cells. GFAP is a marker expressed on astrocytes which are also differentiated from neural stem cells.

The presence of NeuN-positive cells was analyzed using goat anti-NeuN monoclonal antibody (Chemicon, Temecula, USA).The presence of GFAP-positive cells was analyzed using goat anti-GFAP polyclonal antibody (Sigma, St. Louis, USA). The presence of BrdU-positive/GFAP-positive cells was analyzed by double-immunofluorescent stain using a combination of primary antibody of mouse anti-rat BrdU monoclonal antibody and secondary antibody of Rho-labeled rabbit anti-mouse antibody, and a combination of primary antibody of goat anti-GFAP polyclonal antibody and secondary antibody of FITC-labeled rabbit anti-goat antibody.

(8) Analysis of BrdU-Positive (Migrating) Cells

Thirty Rats in each group were intraperitoneally injected twice with 50 mg/kg of BrdU within 24 hours after model established. The rats were autopsied on day 7, 14 and 21 after model established for preparation of frozen brain sections. The presence of BrdU-positive cells in the SVZ region was analyzed by immunochemical stain using mouse anti-rat BrdU monoclonal antibody.

Results and Discussion

(1) Evaluation of Neural Function

The scores were detected on every day after the ischemia/reperfusion injury model established until day 21 of terminal of the experiment.

Table 7. Changes of neural function scores on time course (n=15, median)

| | Day 1 | Day 2 | Day 3 | Day 4 | Day 5 | Day 6 | Day 7 | Day 8 | Day 9 | Day 10 |
|---|---|---|---|---|---|---|---|---|---|---|
| Sham-operation group | 0 | 0 | 0 | 0 | 0 | 0 | 0 | 0 | 0 | 0 |
| Model group | 3 | 3 | 3 | 2 | 2 | 2 | 2 | 2 | 1 | 1 |
| Batroxobin group | 3 | 3 | 2 | 2 | 1 | 1 | 1* | 0* | 0* | 0 |

| | Day 11 | Day 12 | Day 13 | Day 14 | Day 15 | Day 16 | Day 17 | Day 18 | Day 19 | Day 20 | Day 21 |
|---|---|---|---|---|---|---|---|---|---|---|---|
| Sham-operation group | 0 | 0 | 0 | 0 | 0 | 0 | 0 | 0 | 0 | 0 | 0 |
| Model group | 1 | 1 | 1 | 1 | 1 | 1 | 1 | 1 | 1 | 1 | 1** |
| Batroxobin group | 0 | 0 | 0 | 0 | 0 | 0* | 0 | 0 | 0 | 0 | 0** |

*Indicates P<0.05 as compared with the model group, **indicates P<0.05 as compared with the day 1 of each group using the Kruskal-Wallis test.

In the sham-operation group, the neural function disorders were not observed. In the other two groups, significant recovery of neural function was observed on day 21 compared with that on day 1 after model established (P<0.05).

In the batroxobin group, recovery of neural function was observed to occur earlier than that in the model group. Significant recovery of neural function was observed in the batroxobin group particularly on day 7, 8, 9 and 16 after model established compared with the model group.

This result indicates that batroxobin activated neural stem cells and/or neural progenitor cells, resulting in contributed to improvement of neural function disorders. The activating effect of batroxobin is considered to be on promoting proliferation, migration and differentiation of the neural stem cells and/or neural progenitor cells.

(2) Demonstration of the Presence of Neural Stem Cells

Table 8. The numbers of nestin-positive cells in the SVZ region (mean$\pm$SD, No. cells/mm$^2$)

| | Day 7 | Day 14 | Day 21 |
|---|---|---|---|
| Sham-operation group | 42.1$\pm$5.86 | 12.3$\pm$1.58 | 1.97$\pm$0.82 |
| Model group | 209.5$\pm$21.6** | 76.5$\pm$12.3** | 45.1$\pm$5.60** |
| Batroxobin group | 276.3$\pm$28.5*** | 97.2$\pm$13.7 | 59.2$\pm$6.84 |
| **Indicates P<0.01 as compared with the sham-operation group, ***indicates P<0.05 as compared with the model group on the same day using the Wilcoxon's test. | | | |

According to the data shown in Table 8, the relative value (%) of the number of nestin-positive cells in the batroxobin group was calculated based on a value of 100% for the model group using the following formula.

$$BRt(\%)=(Bt\text{-}St)/(Mt\text{-}St)\times 100$$

BRt(%): Percentage of nestin-positive cells in batroxobin group at time t
Bt: Mean number of nestin-positive cells in batroxobin group at time t
St: Mean number of nestin-positive cells in sham-operation group at time t
Mt: Mean number of nestin-positive cells in model group at time t
t: Day 7, day 14 or day 21

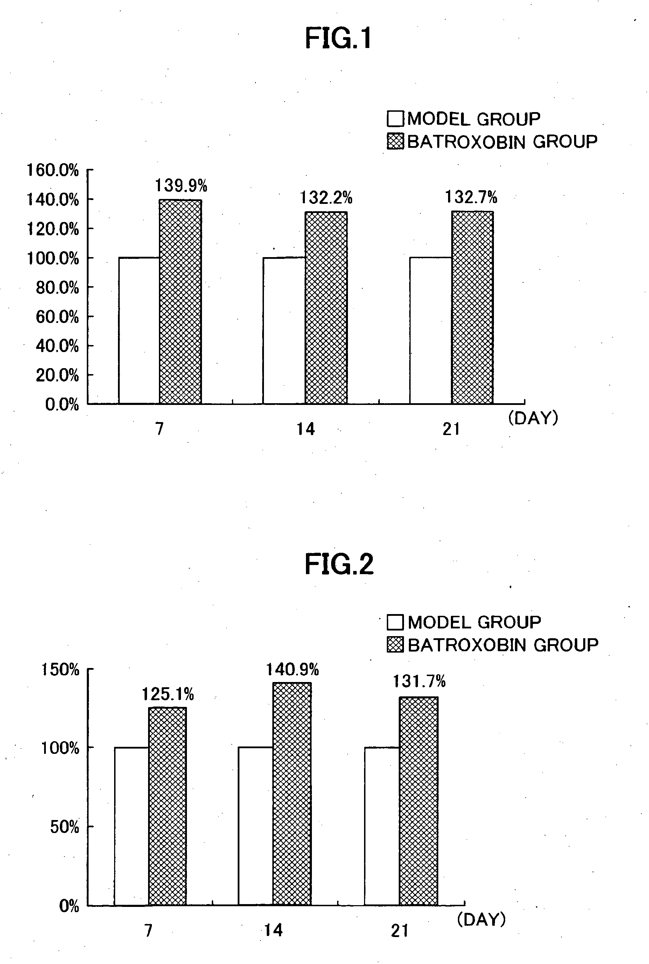

The results are shown in Fig. 1. In comparison with the sham-operation group, the number of nestin-positive cells increased in the model group (nestin is a marker for neural stem cells) (Table 8). This is considered to be attributable to a compensatory reaction induced by stimulation of the cerebral ischemia/reperfusion injury in the animals.

On the other hand, the numbers of nestin-positive cells increased in the batroxobin group as compared with the model group on each of day 7, 14 and 21 after model established (Table 8, Fig. 1).

These results indicate that batroxobin activated the nestin-positive cells (i.e., neural stem cells). The activating effect of batroxobin is considered to be on promoting proliferation and migration of the neural stem cells.

In addition, although batroxobin was not administrated from day 9 after model established, the numbers of nestin-positive cells significantly increased in the batroxobin group as compared with the model group on day 21 after model established (Fig. 1). This result indicates that batroxobin has a sustainable activating effect on neural stem cells.

(3) Demonstration of Presence of Proliferating Neural Stem Cells and Their Self-Replication Ability

1) Demonstration of Presence of Proliferating Neural Stem Cells

The presence of proliferating nestin-positive/BrdU-positive cells (i.e., neural stem cells) were confirmed by double-immunofluorescent stain in the SVZ region in the batroxobin group (data not shown). This indicates the presence of proliferating neural stem cells in the batroxobin group.

2) Demonstration of the Self-Replication Ability of Neural Stem Cells

Table 9. The numbers of BrdU-positive cells in the SVZ region (mean$\pm$SD, No. cells/mm$^2$)

|  | Day 7 | Day 14 | Day 21 |
|---|---|---|---|
| Sham-operation group | 51.7$\pm$3.76 | 32.8$\pm$2.82 | 21.9$\pm$1.87 |
| Model group | 136.45$\pm$9.82** | 327.6$\pm$12.4** | 277.2$\pm$12.5** |
| Batroxobin group | 157.71$\pm$11.3 | 448.2$\pm$27.7*** | 358.6*** |
| **Indicates P<0.01 as compared with the sham-operation group, ***indicates P<0.01 as compared with the model group on the same day using the Wilcoxon's test. | | | |

According to the data shown in Table 9, the relative value (%) of the number of BrdU-positive cells in the batroxobin group was calculated based on a value of 100% for the model group using the following formula.

$$BRt(\%)=(Bt\text{-}St)/(Mt\text{-}St)\times 100$$

BRt(%): Percentage of BrdU-positive cells in batroxobin group at time t
Bt: Mean number of BrdU-positive cells in batroxobin group at time t
St: Mean number of BrdU-positive cells in sham-operation group at time t
Mt: Mean number of BrdU-positive cells in model group at time t
t: Day 7, day 14 or day 21

The results are shown in Fig. 2. In comparison with the sham-operation group, the number of BrdU-positive cells increased in the model group (BrdU is a marker for proliferating cells) (Table 9). This is considered to be attributable to a compensatory reaction induced by stimulation of the cerebral ischemia/reperfusion injury in the animals.

On the other hand, the numbers of BrdU-positive cells increased in the batroxobin group as compared with the model group on each of day 7, 14 and 21 after model established (Table 9, Fig. 2).

BrdU-positive cells present in brain tissue consist only of neural stem cells and neural progenitor cells which are capable of incorporating BrdU based on their self-replication ability. Thus, these results indicate that batroxobin activated neural stem cells and/or neural progenitor cells. The activating effect of batroxobin is considered to be on promoting proliferation and migration of the neural stem cells and/or neural progenitor cells.

In addition, although batroxobin was not administrated from day 9 after model established, the numbers of BrdU-positive cells significantly increased in the batroxobin group as compared with the model group on day 21 after model established (Fig. 2). This indicates that batroxobin has a sustainable activation effect on neural stem cells and/or neural progenitor cells.

(4) Demonstration of Presence of Immature Neurons, Astrocytes Differentiated from Neural Stem Cells and the Astrocytes Increased

1) Demonstration of Presence of Immature Neurons

The presence of NeuN-positive cells was confirmed in the batroxobin group (data not shown). NeuN is a marker expressed on the immature neurons which are differentiated from neural stem cells. Thus, this result indicates that immature neurons differentiated from neural stem cells are present in the batroxobin group.

2) Demonstration of Presence of Astrocytes Differentiated from Neural Stem Cells

The presence of BrdU-positive/GFAP-positive cells was confirmed using double-immunofluorescent stain in the SVZ region in the batroxobin group (data not shown). GFAP is a marker expressed on astrocytes which are differentiated from neural stem cells. Thus, this result indicates that astrocytes differentiated from neural stem cells are present in the batroxobin group. However, astrocytes are differentiated cells in the neural tissues, why BrdU was incorporated into them? It is considered that the detected BrdU is incorporated into the cells when in the state of stem cells and/or progenitor cells prior to differentiating into astrocytes, and existed continuously.

3) Demonstration of Presence of the Astrocytes Increased

Table 10. The numbers of GFAP-positive cells in the SVZ region (mean$\pm$SD, No. of cells/mm$^2$)

|  | Day 7 | Day 14 | Day 21 |
|---|---|---|---|
| Sham-operation group | 2.2$\pm$0.18 | 1.3$\pm$0 | 0 |
| Model group | 66.4$\pm$21.8** | 57.6$\pm$16.4** | 27.7$\pm$12.5** |
| Batroxobin group | 87.1$\pm$26.4*** | 68.2$\pm$17.7 | 34.9$\pm$16.8 |
| **Indicates P<0.01 as compared with the sham-operation group, ***indicates P<0.05 as compared with the model group on the same day using the Wilcoxon's test. | | | |

According to the data shown in Table 10, the relative value (%) of the number of GFAP-positive cells in the batroxobin group was calculated based on a value of 100% for the model group using the following formula.

$$BRt(\%)=(Bt\cdot St)/(Mt\cdot St)\times100$$

BRt(%): Percentage of GFAP-positive cells in batroxobin group at time t

Bt: Mean number of GFAP-positive cells in batroxobin group at time t

St: Mean number of GFAP-positive cells in sham-operation group at time t

Mt: Mean number of GFAP-positive cells in model group at time t

t: Day 7, day 14 or day 21

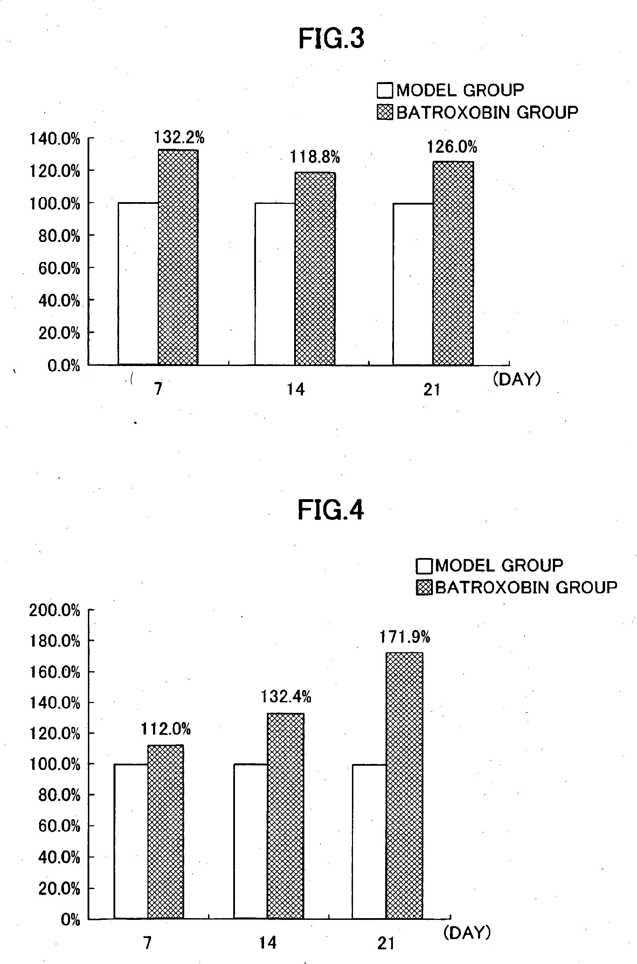

The results are shown in Fig. 3. In comparison with the sham-operation group, the number of GFAP-positive cells increased in the model group (GFAP is a marker for astrocytes differentiated from neural stem cells) (Table 10). This is considered to be attributable to a compensatory reaction induced by the stimulation of cerebral ischemia/reperfusion injury in the animals.

On the other hand, the numbers of GFAP-positive cells increased in the batroxobin group as compared with the model group on each of day 7, 14 and 21 after model established (Table 10, Fig. 3).

These results indicate that batroxobin activated the GFAP-positive cells (i.e., neural stem cells and/or neural progenitor cells). The activating effects of batroxobin is considered to be on promoting proliferation, differentiation and migration of neural stem cells and/or neural progenitor cells, and particularly on promoting differentiation to astrocytes from above cells.

In addition, although batroxobin was not administrated from day 9 after model established, the numbers of GFAP-positive cells increased in the batroxobin group as compared with the model group on day 21 after model established (Fig. 3). This result indicates that batroxobin has a sustainable activating effect on neural stem cells and/or neural progenitor cells to differentiate into astrocytes.

(5) <u>Demonstration of Presence of the Migrating Cells</u>

Table 11. The numbers of BrdU-positive cells in the SVZ region (mean$\pm$SD, No. cells/mm$^2$)

|  | Day 7 | Day 14 | Day 21 |
|---|---|---|---|
| Sham operation group | 2.25$\pm$1.08 | 3.75$\pm$1.33 | 3.14$\pm$1.09 |
| Model group | 122.3$\pm$14.2** | 78.5$\pm$8.3** | 33.6$\pm$5.37** |
| Batroxobin group | 136.7$\pm$15.3 | 102.7$\pm$11.2*** | 55.5$\pm$8.23 |
| **Indicates P<0.01 as compared with the sham-operation group, ***indicates P<0.05 as compared with the model group on the same day using the Wilcoxon's test. | | | |

According to the data in Table 11, the relative value (%) of the number of BrdU-positive cells in the batroxobin group was calculated based on a value of 100% for the model group using the following formula.

$$BRt(\%) = (Bt\text{-}St)/(Mt\text{-}St) \times 100$$

BRt(%): Percentage of BrdU-positive cells in batroxobin group at time t
Bt: Mean number of BrdU-positive cells in batroxobin group at time t
St: Mean number of BrdU-positive cells in sham-operation group at time t
Mt: Mean number of BrdU-positive cells in model group at time t
t: Day 7, day 14 or day 21

The results are shown in Fig. 4. In this experiment, BrdU was intraperitoneally administered only twice within 24 hours after model established, and since BrdU was not administered after that time, the BrdU-positive cells analyzed on day 7, 14 and 21 after model established are the BrdU-positive cells presented at the BrdU-administered time. Thus, increases in the number of BrdU-positive cells analyzed in the SVZ region on day 7, 14 and 21 after model established reflect the number of BrdU-positive cells which have migrated into the SVZ region. Moreover, BrdU-positive cells in the SVZ region consist only of neural stem cells and neural progenitor cells capable of incorporating BrdU based on the self-replication ability. On the basis of the above; Table 11 and Fig. 4 indicate the migration status of neural stem cells and neural progenitor cells into the SVZ region.

In comparison with the sham-operation group, the number of BrdU-positive cells in the model group increased (Table 11). This is considered to be attributable to a compensatory reaction induced by stimulation of the cerebral ischemia/reperfusion injury in the animals.

On the other hand, the numbers of BrdU-positive cells increased in the batroxobin group as compared with the model group on day 7, 14 and 21 after model established (Table 11, Fig. 4). This indicates that the number of neural stem cells and/or neural progenitor cells which migrated to the SVZ region in the batroxobin group was larger than in the model group.

These results indicate that batroxobin activated neural stem cells and/or neural progenitor cells. The activating effect of batroxobin is considered to be on promoting migration of the neural stem cells and/or neural progenitor cells.

In addition, although batroxobin was not administrated from day 9 after model established, the numbers of BrdU-positive cells increased in the batroxobin group as compared with the model group even on day 21 after model established(Fig. 4). This result indicates that batroxobin has a sustainable activating effect on migration of neural stem cells and/or or neural progenitor cells.

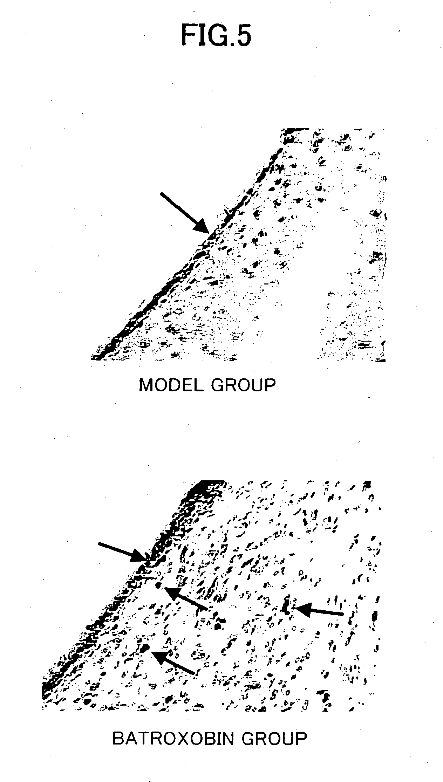

Furthermore, the distribution of BrdU-positive cells in SVZ region was compared between the model group and batroxobin group by immunochemical stain. In the model group, BrdU-positive cells were only distributed on the surface of the SVZ region (left side of Fig. 5), but the BrdU-positive cells were widely distributed from the surface to interior of the SVZ region in the batroxobin group (right side of Fig. 5). This indicates that neural stem cells and/or neural progenitor cells migrated from the surface to the interior of the SVZ region as a result of administration of batroxobin, namely that batroxobin promoted migration of neural stem cells and/or neural progenitor cells in situ of the brain tissue.

These results in Example 3 clearly indicate that batroxobin has sustainable activating effects on neural stem cells and/or neural progenitor cells. The activating effects of batroxobin are considered to be on promoting proliferation, migration and differentiation of the neural stem cells and/or neural progenitor cells.

INDUSTRIAL APPLICABILITY

As described in the examples above, an activating agent of the present invention is capable of activating stem cells and/or progenitor cells. Thus, the agent can be advantageously used in regenerative medicine, and particularly in regenerative medicine utilizing self-regeneration.

Since regenerative medicine utilizing self-regeneration carried out by using the activating agent uses stem cells and/or progenitor cells present in the body of the patient, it is not necessary to introduce cells from outside of the patient's body. Thus, the burden on the patient is less and the risk is also absent such as infection occurring during cell implantation, resulting the regenerative medicine utilizing self-regeneration superior to regenerative medicine in which cells are introduced from outside of the patient's body.

In addition, in regenerative medicine utilizing self-regeneration carried out by using the activating agent activation of stem cells and/or progenitor cells occurs specifically at the affected site (i.e., the injured organs and/or tissues) in the patient, and that regeneration only occurs depend on the degree demanded. Thus, the regenerative medicine utilizing self-regeneration has no risk of the occurrence of complications caused by excessive regeneration and/or excessive repair by cell implantation as a mainstream method of conventional regenerative medicine in which cells are introduced from outside of the patient's body.

Moreover, the activating agent comprising a thrombin-like enzyme has few or no side effects, is able to act promptly and moderately depending on the state of advancement and the degree of injured organs and/or tissues to which regenerative medicine is applied, and has sustainable activating effects. Thus, it can be used advantageously in regenerative medicine.

Therefore, the present invention can be used in regenerative medicine with a high industrial applicability.

**Claims**

1. An *in vitro* method for activating CD34-expressing cells by administration of batroxobin.

2. The method according to claim 1, wherein CD34-expressing cells are stem cells and/or progenitor cells.

3. The method according to claim 1, wherein the CD34-expressing cells are mesenchymal stem cells and/or vascular endothelial progenitor cells.

**Patentansprüche**

1. *In vitro*-Verfahren zum Aktivieren von CD34-exprimierenden Zellen durch Verabreichung von Batroxobin.

2. Verfahren nach Anspruch 1, wobei die CD34-exprimierenden Zellen Stammzellen und/oder Vorläuferzellen sind.

**3.** Verfahren nach Anspruch 1, wobei die CD34-exprimerenden Zellen mesenchymale Stammzellen und/oder vaskuläre endotheliale Vorläuferzellen sind.

**Revendications**

**1.** *Procédé in vitro* pour activer des cellules exprimant CD34 par administration de batroxobine.

**2.** Procédé selon la revendication 1, dans lequel les cellules exprimant CD34 sont des cellules souches et/ou des cellules progénitrices.

**3.** Procédé selon la revendication 1, dans lequel les cellules exprimant CD34 sont des cellules souches mésenchymateuses et/ou des cellules progénitrices endothéliales vasculaires.

## FIG.1

☐MODEL GROUP
▨BATROXOBIN GROUP

## FIG.2

☐MODEL GROUP
▨BATROXOBIN GROUP

## FIG.3

☐ MODEL GROUP
▨ BATROXOBIN GROUP

## FIG.4

☐ MODEL GROUP
▨ BATROXOBIN GROUP

# FIG.5

MODEL GROUP

BATROXOBIN GROUP

EP 1 929 006 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 55710718 B

- JP 1118129 A

### Non-patent literature cited in the description

- **BARRIERS.** *Science,* 2000, vol. 287, 1442-1446
- **MCCLOSKEY KE et al.** Use of embryonic stem cell-derived endothelial cells as a cell source to generate vessel structures in vitro. *Tissue Eng,* 2005, vol. 11, 497-505
- **SATO Y et al.** Can a bone marrow cell contribute to organ regeneration? In vivo analysis using transgenic rats with reporter genes. *Transplantation Proceedings,* 2005, vol. 37, 273-275
- **GARRY DJ et al.** Ponce de Leon's fountain stem cells and the regenerating heart. *Am J Med Sci,* 2005, vol. 329 (4), 190-201
- **GARRY DJ et al.** Ponce de leon's fountain: stem cells and the regenerating heart. *Am J Med Sci,* 2005, vol. 329 (4), 190-201
- **REYNOLDS BA ; WEISS S.** Generation of neurons and astrocytes from isolated cells of the adult mammalian central nervous system. *Science,* 1992, vol. 255, 1707-1710
- **SHIHABUDDIN LS et al.** Adult spinal cord stem cells generate neurons after transplantation in the adult dentate gyrus. *The J Neuroscience,* 2000, vol. 20, 8727-8735
- **SHEN Q et al.** Endothelial cells stimulate self-renewal and expand neurogenesis of neural stem cells. *Science,* 2004, vol. 304, 1338-1440
- **TERAMOTO T et al.** EGF amplifies the replacement of parvalbumin-expressing striatal interneurons after ischemia. *The J Clinical Investigation,* 2003, vol. 111, 1125-1132
- **WALTER DH et al.** Statin therapy accelerates reendothelialization: A novel effect involving mobilization and incorporation of bone marrow-derived endothelial progenitor cells. *Circulation,* 2002, vol. 105, 3017-3024
- **TAKEYAMA K ; OHTO H.** PBSC mobilization. *Transfus Apher Sci,* 2004, vol. 31, 233-243
- **AICHER A ; ZEIHER AM ; DIMMELER S.** Mobilizing endothelial progenitor cells. *Hypertension,* 2005, vol. 45, 321-325
- Encyclopedia of Immunology. Tokyo Kagaku Dozin, 03 December 2001, 327

- Snake venom proteins affecting hemostasis and fibrinolysis. **STOCKER KF.** Medical Use of Snake Venom Proteins. CRC Press, 1990, 130-131
- **ARONSON DL.** Comparison of the actions of thrombin and the thrombin-like venom enzymes Ancrod and Batroxobin. *Thrombos Haemostas,* 1976, vol. 36, 9-13
- **ITOH N et al.** Molecular cloning and sequence analysis of cDNA for batroxobin, a thrombin-like snake venom enzyme. *J Biol Chem,* 1987, vol. 262, 3132-3135
- Snake venom proteins affecting hemostasis and fibrinolysis. **STOCKER KF.** Medical Use of Snake Venom Proteins. CRC Press, 1990, 134-135
- Snake venom proteins affecting hemostasis and fibrinolysis. **STOCKER KF.** Medical Use of Snake Venom Protein. CRC Press, 1990, 140-141
- **MCKAY R.** Stem cells hype and hope. *Nature,* 2000, vol. 406, 361-364
- **GOULD E ; GROSS CG.** Neurogenesis in adult mammals: some progress and problems. *The J Neuroscience,* 2002, vol. 22 (3), 619-623
- **WIESE, C. et al.** Nestin expression - a property of multi-lineage progenitor cells?. *Cellular and Molecular Life Sciences,* 2004, vol. 61, 2510-2522
- **ASAHARA T et al.** Isolation of putative progenitor endothelial cells for angiogenesis. *Science,* 1997, vol. 275, 964-967
- **SHI Q et al.** Evidence for circulating bone marrow-derived endothelial cells. *Blood,* 1998, vol. 92 (2), 362-367
- **OZAKI M et al.** Toxicity of the defibrase, Defibrinogen Batroxobin (First Report). *Pharmacometrics,* 1983, vol. 25, 339-346
- **MUROHARA T et al.** Transplanted cord blood-derived endothelial precursor cells augment postnatal neovascularization. *J Clin Invest,* 2000, vol. 105, 1527-1536
- **MICHEJDA M.** Which stem cells should be used for transplantation?. *Fetal Diagn Ther,* 2004, vol. 19, 2-8

- **ZHAO Y et al.** A human peripheral blood monocyte-derived subset acts as pluripotent stem cells. *Proc Natl Acad Sci USA,* 2003, vol. 100, 2426-2431
- **FADINI GP et al.** Circulating endothelial progenitor cells are reduced in peripheral vascular complications of tape 2 diabetes mellitus. *J American College of Cardiology,* 2005, vol. 45, 1449-1457
- **KUWANA M et al.** Human circulating CD14+ monocytes as a source of progenitors that exhibit mesenchymal cell differentiation. *J Leukoc Biol,* 2003, vol. 74, 833-845
- **HRISTOV M ; WEBER C.** Endothelial progenitor cells: characterization, pathophysiology, and possible clinical relevance. *J Cell Mol Med,* 2004, vol. 8, 498-508
- **LONGA EZ et al.** Reversible middle cerebral artery occlusion without craniectomy in rats. *Stroke,* 1989, vol. 20, 84-91